# EUROPEAN PATENT APPLICATION

(11) **EP 3 404 359 A1**
(43) Date of publication of application: **21.11.2018**
(21) Application number: 18172763.7
(22) Date of filing: 16.05.2018
(51) Int. Cl.: G01B 11/25, H04N 13/254

(54) **PATIENT MONITORING SYSTEM**

(30) Priority: 17.05.2017 GB 201707910
(71) Applicant: Vision RT Limited, London N3 2JU (GB)
(72) Inventor: HALE, Gideon Matthew, London, N3 2JU (GB); HANSON, Peter John, London, N3 2JU (GB)
(74) Representative: William Demant

(57) **Abstract**

A patient monitoring system for monitoring the location of a patient (20) at a distance is described, comprising a projector (52) operable to project a pattern of light onto the surface of a patient (20) and a imaging system (46,50) operable to obtain images of a patient (20) on to whom a pattern of light is projected. A heat sink (55) is associated with the projector (52). A heat source (74), such as an array of resistors, is configured to apply heat to the heat sink (55) when the projector (52) is not being operated, reducing variation in the temperature of the heat sink (55) which in turn reduces variation in thermal expansion and contraction of the monitoring system which can be a potential source of error for determining the position of a patient (20) being monitored.

## Description

The present application concerns a patient monitoring system. More specifically the present application concerns a patient monitoring system for monitoring the positioning and movement of patients. The invention is particularly suitable for use with radio therapy devices and computed tomography (CT) scanners and the like where highly accurate positioning and the detection of patient movement and breathing is important for successful treatment.

A number of patient monitoring systems for monitoring a patient's movement and breathing during radiotherapy and scanning have been proposed to assist with patient positioning and monitoring. These include Vision RT's patient monitoring system which has previously been described in U.S. Pat. No. 7,348,974, U.S. Pat. No. 7,889,906 and U.S. Pat. No. 9,028,422, all of which are hereby incorporated by reference. In the systems described in Vision RT's patent applications, stereoscopic images of a patient are obtained and processed to generate data identifying 3D positions of a large number of points corresponding to points on the surface of an imaged patient. Such surface data can be compared with data generated on a previous occasion and used to position a patient in a consistent manner or provide a warning when a patient moves out of position.

Very small changes in relative position between image detectors which arise due to external temperature variations can result in pixel drift which reduces the accuracy of a stereoscopic camera based patient monitoring system. Such changes naturally occur as the image sensors and their mountings expand and contract with variations in temperature. Although such movements are very small, they are such that the calibration of sensors can become misaligned so that portions of an image are registered in adjacent pixels. Where an image sensor is imaging a patient at a distance this very small change in location is registered as a much larger change in the position of the portion of the surface of the patient being monitored.

To address such a problem, Vision RT proposed in US Patent Application No. 2015/0062303, providing a stereoscopic camera where the camera is configured to maintain the image detectors at a temperature above ambient room temperature thereby substantially isolating the image detectors within a camera from external variations in temperature. This then reduces variations in the relative positions and viewpoints of the image detectors due to thermal contraction and expansion. Adopting such an approach Vision RT have been able to improve the accuracy their monitoring systems so that such systems are able to locate the position of a patient within 0.5 mm.

Further improvements in accuracy are, however, still desirable.

In accordance with one aspect of the present invention there is provided a patient monitoring system for monitoring the location of a patient at a distance, comprising: a projector operable to project a pattern of light onto the surface of a patient; an imaging system operable to obtain images of a patient on to whom a pattern of light is projected; a heat sink associated with the projector; and a heat source configured to apply heat to the heat sink when the projector is not being activated. Applying heat to a heat sink when the projector is not being activated reduces the variation of the temperature of the heat sink and hence reduces the variation in thermal contraction and expansion of the monitoring system as a whole. Adopting this approach Vision RT have improved the accuracy of their monitoring systems so that their systems are able to locate the position of a patient to within 0.15 mm.

The projector may comprise a projector operable to project a speckle pattern onto the surface of a patient. In such an embodiment the projector may comprise a light source such as an LED light source wherein light generated by the light source is projected onto the surface of a patient via a film on which a speckle pattern is provided. Alternatively the projector may comprise a projector operable to project structured light such as a grid pattern or a line of light on the surface of a patient. Where a projector is arranged to project structured light onto the surface of a patient, the light source may comprise a laser light source.

In embodiments of the present invention, the heat source may comprise a resistor or an array of resistors. The resistance of the resistor(s) may be selected on the basis of the power consumption of the light source of the projector so that operation of the heat source and/or the light source substantially generates the same amount of heat. Thus when the light source of the projector and the resistor(s) are operated in anti-phase, a substantially constant amount of heat is applied to the heat sink.

In some embodiments, the monitoring system may include a thermometer operable to measure the temperature of the heat sink and the heat source may be responsive to the thermometer to apply heat to the heat sink when the projector is not being activated so as to maintain the heat sink at a substantially constant temperature.

The projector, imaging system and heat source may all be provided inside a housing where the heat sink protrudes through the housing and is arranged to transfer heat from within the housing to the exterior of the housing. Vents maybe provided in the housing to permit air within said housing to exit said housing. A fan may be provided for driving air through the housing and expelling air out of the housing via the vents.

The imaging system may comprise a stereoscopic camera in which a pair of image detectors are provided mounted in a fixed relationship relative to one another. Alternatively, the imaging system may comprise a single image detector. The image detector(s) of the imaging system may be associated with a heating system arranged to maintain the image detectors at a substantially constant temperature.

A processing system may be provided operable to process image data obtained by the imaging system and generate a 3D computer wire mesh model of the surface of a patient imaged by imaging system. Such a processing system may be operable to compare a generated model with a stored model surface.

The monitoring system may be utilized in conjunction with a treatment apparatus including a mechanical couch operable to position a patient in accordance with instructions generated by the processing system. In some such embodiments the monitoring system may be arranged to generate a warning signal when the surface of a patient is identified as being out of position by more than a threshold amount relative to a stored model surface of the patient. Alternatively in some embodiments the monitoring system may be arranged to activate or inhibit a treatment apparatus on the basis of a comparison between a generated model surface and a stored model surface.

### BRIEF DESCRIPTION OF THE DRAWINGS

An embodiment of the present invention will now be described with reference to the accompanying drawings in which:
Figure 1 is a schematic diagram of a patient monitoring system in accordance with the present invention;
Figure 2 is a schematic perspective view of the exterior of the stereoscopic camera of the monitoring system of Figure 1; and
Figure 3 is a schematic block diagram of the interior of the stereoscopic camera of the monitoring system of Figure 1.

### SPECIFIC EMBODIMENT

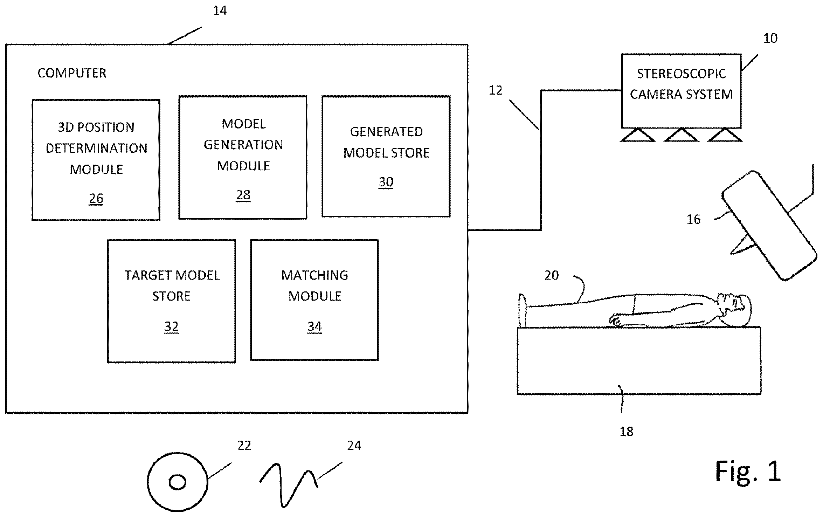

Figure 1 is a schematic diagram of a patient monitoring system in accordance with an embodiment of the present invention. The patient monitoring system in this embodiment comprises a stereoscopic camera 10 connected by wiring 12 to a computer 14. The computer 14 is also connected to treatment apparatus 16 such as a linear accelerator for applying radiotherapy or an x-ray simulator for planning radiotherapy. A mechanical couch 18 is provided as part of the treatment apparatus upon which a patient 20 lies during treatment. The treatment apparatus 16 and the mechanical couch 18 are arranged such that under the control of the computer 14 the relative positions of the mechanical couch 18 and the treatment apparatus 16 may be varied, laterally, vertically, longitudinally and rotationally.

In use, the stereoscopic camera 10 obtains video images of a patient 20 lying on the mechanical couch 18. These video images are passed via the wiring 12 to the computer 14. The computer 14 then processes the images of the patient 20 to generate a model of the surface of the patient 20. This model is compared with a model of the patient 20 generated during earlier treatment sessions. When positioning a patient 20 the difference between a current model surface and a target model surface obtained from an earlier session is identified and the positioning instructions necessary to align the surfaces determined and sent to the mechanical couch 18. Subsequently during treatment any deviation from an initial set up can be identified and if the deviation is greater than a threshold, the computer 14 sends instructions to the treatment apparatus 16 to cause treatment to be halted until a patient 20 can be repositioned.

In order for the computer 14 to process images received from the stereoscopic camera 10, the computer 14 is configured by software either provided on a disk 22 or by receiving an electrical signal 24 via a communications network into a number of functional modules 26-34. It will be appreciated that the functional modules 26-34 illustrated in Figure 1 are purely notional in order to assist with the understanding of the working of the claimed invention and may not in certain embodiments directly correspond with blocks of code in the source code for the software. In other embodiments the functions performed by the illustrated functional modules 26-34 may be divided between different modules or may be performed by the re-use of the same modules for different functions.

In this embodiment, the functional modules 26-34 comprise: a 3D position determination module 26 for processing images received from the stereoscopic camera 10, a model generation module 28 for processing data generated by the 3D position determination module 26 and converting the data into a 3D wire mesh model of an imaged surface; a generated model store 30 for storing a 3D wire mesh model of an imaged surface; a target model store 32 for storing a previously generated 3D wire mesh model; and a matching module 34 for determining rotations and translations required to match a generated model with a target model.

In use, as images are obtained by the stereoscopic camera 10, these images are processed by the 3D position determination module 26 to identify 3D positions of corresponding points in pairs of images. This is achieved by the 3D position determination module 26 identifying corresponding points in pairs of images obtained by the stereoscopic camera 10 and then determining 3D positions for those points based on the relative positions of corresponding points in obtained pairs of images and stored data identifying the relative positions of cameras obtaining the images.

Typically the identification of corresponding points is based on analysis of image patches of around 16x16 pixels. In order to assist with identifying and matching corresponding patches as will be described, the stereoscopic camera 10 is arranged to project a random or quasi random speckle pattern onto the patient 20 being imaged so that different portions of the surface of the patient 20 can be more easily distinguished. The size of the speckle pattern is selected so that different patterns will be apparent in different image patches.

The position data generated by the 3D position determination module 26 is then passed to the model generation module 28 which processes the position data to generate a 3D wire mesh model of the surface of a patient 20 imaged by the stereoscopic cameras 10. In this embodiment the 3D model comprises a triangulated wire mesh model where the vertices of the model correspond to the 3D positions determined by the 3D position determination module 26. When such a model has been determined it is stored in the generated model store 30.

When a wire mesh model of the surface of a patient 20 has been stored, the matching module 34 is then invoked to determine a matching translation and rotation between the generated model based on the current images being obtained by the stereoscopic camera 10 and a previously generated model surface of the patient stored in the target model store 32.

The determined translation and rotation can then be sent as instructions to the mechanical couch 18 to cause the couch to position the patient 20 in the same position relative to the treatment apparatus 16 as they were when they were previously treated. Subsequently, the stereoscopic camera 10 can continue to monitor the patient 20 and any variation in position can be identified by generating further model surfaces and comparing those generated surfaces with the target model stored in the target model store 32. If it is determined that a patient has moved out of position, the treatment apparatus 16 can be halted and the patient 20 repositioned, thereby avoiding irradiating the wrong parts of the patient 20.

In some embodiments, the treatment apparatus 16 may be activated, for example to apply radiation to a patient 20 when the generated models surface corresponds with a stored model surface within a certain tolerance. In that way radiation may be applied to a patient 20 at a fixed point within the breathing cycle and thereby applied when a thoracic tumor or other tumor subject to movement during the breathing cycle may be assumed to be at a particular location within the body.

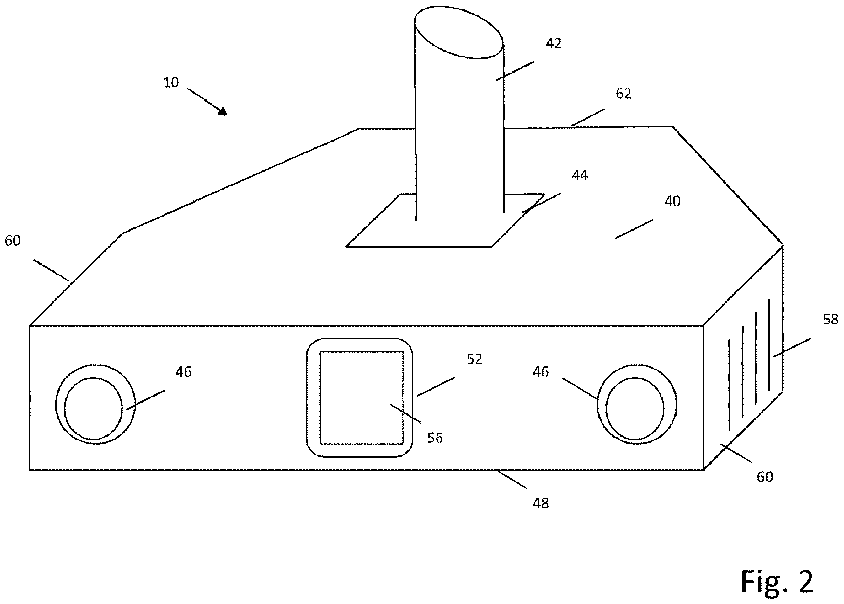

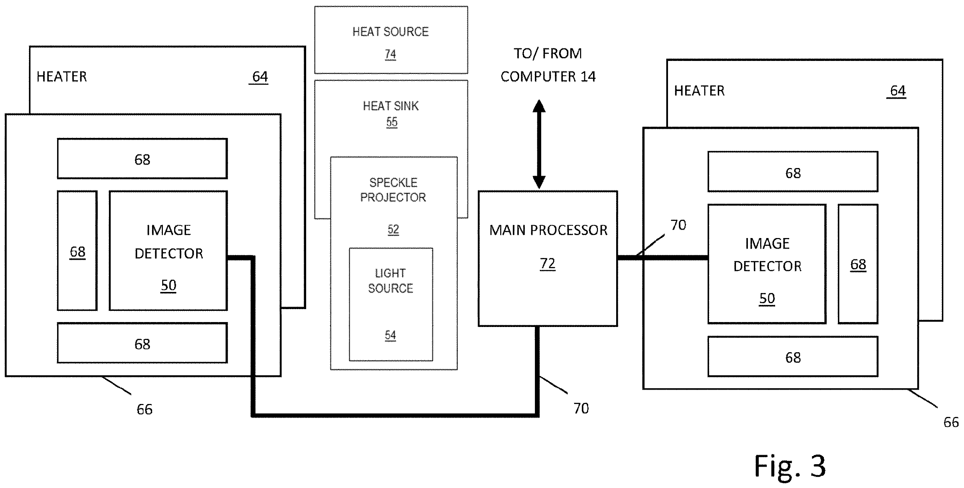

Figure 2 is a schematic perspective view of the exterior of the stereoscopic camera 10 and Figure 3 is a schematic block diagram of the interior of the stereoscopic camera 10 of Figure 1.

In this embodiment the stereoscopic camera 10 comprises a housing 40 which is connected to a bracket 42 via a hinge 44. The bracket 42 enables the stereoscopic camera 10 to be attached in a fixed location to the ceiling of a treatment room whilst the hinge 44 permits the orientation of the stereoscopic camera 10 to be orientated relative to the bracket 42 so that the stereoscopic camera 10 is arranged to view a patient 20 on a mechanical couch 18.

A pair of lenses 46 are mounted at either end of the front surface 48 of the housing 40. These lenses 46 are positioned in front of image detectors 50 contained within the housing 40. In this embodiment the image detectors 50 (not shown in Figure 2) comprise CMOS active pixel sensors. In other embodiments charge coupled devices could be used. The image detectors 50 are arranged behind the lenses 46 so as to capture images of a patient 20 via the lenses 46.

A speckle projector 52 is provided in the middle of the front surface 48 of the housing 40 between the two lenses 46. The speckle projector 52 includes a light source 54 (not shown in Figure 2) which in this embodiment comprises a 10W red LED light. The light source 54 is associated with a heat sink 55 (not shown in Figure 2) which transfers heat generated by the light source 54 from within the housing 40 to the exterior of the housing 40.

The speckle projector 52 is arranged to illuminate a patient 20 with a non-repeating speckled pattern of infrared light so that when images of a patient 20 are captured by the two image detectors corresponding portions of captured images can be distinguished. To that end light from the light source 54 is directed via a film 56 with a random speckle pattern printed on the film 56. As a result a pattern consisting of light and dark areas is projected onto the surface of a patient 20.

A series of vents 58 are provided in the side walls 60 of the housing 40. Further vents (not shown) are provided in the rear 62 of the housing 40. A fan (not shown) connected to a temperature sensor (also not shown) is contained within the housing 40.

The temperature sensor is arranged to monitor the ambient temperature within the interior of the housing 40 and if this varies to activate the fan to draw air in via the vents 58 in the side walls 60 of the housing 40 and expel the air via the vents at the rear 62 of the housing 40. In this way an air flow of air at room temperature is caused to circulate within the housing 40 and maintain the interior of the housing 40 at a substantially constant temperature.

Variations in temperature cause small variations in the position of the image detectors 50. Due to the sensitivity of the image detectors such changes do not have to be large in order for one part of an image corresponding to a pixel to be registered at a different pixel. Thus for example it has been determined that movements as small as 2.5 micrometers may cause one part of an image to be registered in an adjacent pixel. When this occurs the accuracy with which 3D positions of points imaged by the image detectors 50 declines.

In this embodiment a heater 64 is provided attached to the rear of the circuit board 66 on which each of the image detectors 50 is mounted. Additionally on the circuit board a series of copper conducting pads 68 are provided surrounding the image detector 50 except on one side enabling wiring 70 to connect the image detectors 50 to a main processor 72. When the heaters 64 are arranged to heat to a temperature above ambient room temperature the effect of the heaters 64 and the conducting pads 68 is to substantially isolate the image detectors 50 from external variations in temperature. Thus effectively the image detectors 50 are enclosed in their own constant temperature micro-climates.

To reduce temperature variations within the housing 40, immediately adjacent the image detectors, the vents 58 are provided in the side walls of the housing 40 slightly removed from the sensors 50 so that when air is drawn into the interior of the housing 40, it is not drawn past the sensors 50. Rather the fan and temperature sensor are utilized to maintain the temperature of the main body of air within the interior of the housing 40 at a substantially constant level with the air adjacent the sensors 50 being kept at a constant temperature through passive communication and convection with this main body of air. By separating the vents 58 from the sensors 50, the activation and deactivation of the fan does not cause sudden changes in temperature adjacent the sensors 50.

This is particularly the case where as far as possible the image detectors 50 are removed from other external heat sources. In the described embodiment this is achieved by for example using a red LED as a light source 54 for the speckle protector 52 thus reducing internal heating which occurs when for example an iridescent light bulb is used as less power is required to generate the required light levels and further as the generated light is only generated in a relatively narrow wave band it is not necessary to include a colored film to remove light at other wave bands which acts to reflect light and heat back within the body of the housing 40.

In addition, the arrangement of the vents 58 adjacent the image detectors 50 and the direction of airflow away from the detectors 50 and out through the rear 62 of the housing 40 also adds to the extent to which the image detectors 50 can be maintained at a constant temperature as a constant stream of air at ambient temperature passes by the detectors 50 and heating arising from the workings of the stereoscopic camera 10 is ejected from the rear 62 of the housing 40. The detectors 50 are thereby largely shielded from outside heat sources and remain at a constant temperature as determined by the heaters 64.

Although the use of an LED rather than an iridescent light bulb as a light source 54 reduces power consumption and hence internal heating within the housing 40, the light source 54 remains a significant source of heat in the stereoscopic camera 10. The applicants have appreciated that the accuracy of a stereoscopic camera 10 can be improved by associating the light source 54 with a heat sink 55 to dissipate the heat generated by the light source 54 and additionally providing a heat source 74 in the form of an array of resistors arranged to be driven in anti-phase with the light source 54. The resistances of the resistors are selected so that the heat generated by the resistors is largely equivalent to the heat generated by the light source 54 when the light source 54 is activated. Thus in this way by associating the light source 54 with a heat sink 55 and driving a heat source 74 in anti-phase to the activation of the light source 54, the temperature of the heat sink 55 is kept broadly constant. By associating the heat sink 55 with a heat source 74 and driving the heat source 74 in anti-phase to the light source 54 the applicants have discovered that the accuracy of a monitoring device monitoring a patient from a distance of approximately 1.5 meters can be improved from around 0.5mm to around 0.15 mm.

Although in the above described embodiment, a stereoscopic camera 10 including speckle projector 52 which in turn includes an LED light source has been described, it will be appreciated that in other embodiments alternative light sources such as a halogen lamp might be used.

Although in the above described embodiment a system has been described where a temperature sensor and a fan are included within the body of the system in some embodiments the sensor and/or the fan could be omitted.

Although in the above described embodiment, a system has been described where a heat source 74 in the form of an array of resistors is provided, it will be appreciated that in other embodiments the heat source 74 could be in the form of a single resistor.

In the above described embodiment, a patient monitoring system has been described in which a heat source 74 in the form of an array of resistors is provided where the resistances of the resistors are selected so that the heat generated by the resistors is largely equivalent to the heat generated by the light source 54 and the resistors are driven in anti-phase to the light-source 54.

It will be appreciated that in other embodiments the heat source 74 does not necessarily have to be driven in anti-phase to the light source 54 and the heat generated by the heat source 74 does not necessarily have to match the heat generated by the light source 74. Rather in some embodiments it may be sufficient to monitor the temperature of the heat sink 55 and for the heat source 74 to be arranged to apply heat to the heat sink 55 when the light source 54 is off so as to maintain the heat sink at a substantially constant temperature.

Although in the above embodiment a patient monitoring system including a stereoscopic camera 10 operable to obtain images of a speckle pattern projected onto the surface of a patient has been described, it will be appreciated that the present invention is also applicable to other forms of patient monitoring system.

More specifically, in other embodiments, rather than providing a pair of image detectors 50 and a speckle projector 52, a monitoring system could be provided with only a single image detector 50. In such a system rather than projecting a speckle pattern onto the surface of a patient 20, a projector could be arranged to project structured light such as a grid pattern or a line of light onto the surface of a patient 20 and the processor 72 could be arranged to process images to detect distortion of the grid pattern or the line of light to determine the location of the surface of a patient 20. As with the above described embodiment, in such a system the light source for such a system such as a laser light source could be associated with a heat sink 55 and a heat source 74 such as one or more resistors could be provided and driven when the light source 54 is off such that the heat sink 55 was kept at a broadly constant temperature.

## Claims

1. A patient monitoring system for monitoring the location of a patient at a distance, the system comprising:
a projector operable to project a pattern of light onto the surface of a patient;
an imaging system operable to obtain images of a patient on to whom a pattern of light is projected;
a heat sink associated with the projector; and
a heat source configured to apply heat to the heat sink when the projector is not being activated so as to reduce variation of the temperature of the heat sink.

2. A patient monitoring system in accordance with claim 1, wherein the heat source comprises a resistor or an array of resistors.

3. A patient monitoring system in accordance with claim 1 or claim 2, wherein the heat source is configured to apply heat to the heat sink in anti-phase to the operation of the projector.

4. A patient monitoring system in accordance with claim 1 or claim 2, further comprising a thermometer operable to measure the temperature of the heat sink, wherein the heat source is responsive to the thermometer to apply heat to the heat sink when the projector is not being activated so as to maintain the heat sink at a substantially constant temperature.

5. A patient monitoring system in accordance with any preceding claim, wherein the projector is operable to project a speckle pattern onto the surface of a patient.

6. A patient monitoring system in accordance with claim 5, wherein the projector comprises a light source wherein light generated by the light source is projected onto the surface of a patient via a film on which a speckle pattern is provided.

7. A patient monitoring system in accordance with claim 6, wherein the light source comprises an LED light source.

8. A patient monitoring system in accordance with any of claims 1-4, wherein the projector is operable to project structured light on the surface of a patient.

9. A patient monitoring system in accordance with claim 8, wherein the projector comprises a laser light source.

10. A patient monitoring system in accordance with any preceding claim wherein said projector, said imaging system and said heat source are all provided within a housing, and said heat sink is operable to transfer heat from within the housing to the exterior of the housing.

11. A patient monitoring system in accordance with claim 10 wherein vents are provided in said housing which permit air within said housing to exit said housing.

12. A patient monitoring system in accordance with claim 11 further comprising a fan operable to drive air through said housing and expel air via said vents.

13. A patient monitoring system in accordance with any preceding claim, wherein said imaging system comprises one or more image detectors, wherein each of said image detectors is associated with a heater operable to contain the image detector in a micro climate of substantially constant temperature and wherein the imaging system optionally comprises a stereoscopic camera.

14. A patient monitoring system in accordance with any preceding claim, further comprising a processing system operable to process image data obtained by the imaging system and generate a 3D computer wire mesh model of the surface of a patient imaged by the imaging system.

15. A patient monitoring system in accordance with claim 14, wherein said processing system is operable to compare a generated model with a stored model surface.
